**Europäisches Patentamt**

**European Patent Office**

**Office européen des brevets**

(11) Veröffentlichungsnummer: **0 074 477**
**B1**

(12) **EUROPÄISCHE PATENTSCHRIFT**

(45) Veröffentlichungstag der Patentschrift:
**25.11.87**

(21) Anmeldenummer: **82106506.7**

(22) Anmeldetag: **19.07.82**

(51) Int. Cl.⁴: **G 01 N 33/18**, G 01 N 21/33,
B 01 J 23/89

(54) **Bestimmung von Nitrat im Wasser.**

(30) Priorität: **14.09.81 DE 3136363**

(43) Veröffentlichungstag der Anmeldung:
**23.03.83 Patentblatt 83/12**

(45) Bekanntmachung des Hinweises auf die Patenterteilung:
**25.11.87 Patentblatt 87/48**

(84) Benannte Vertragsstaaten:
**AT BE CH DE FR GB LI NL**

(56) Entgegenhaltungen:
**DE-A-2 460 078**
**GB-A-1 328 532**
**US-A-2 822 475**
**US-A-2 967 835**
**US-A-3 228 991**
**US-A-4 083 809**

**CHEMICAL ABSTRACTS**, Band 83, Nr. 4, 28. Juli
1975, Seite 288, Nr. 32667c, Columbus, Ohio, US; J.C.
MOORE: "New direct ultraviolet method for the
analysis of nitrate ions" & EFFLUENT WATER
TREAT. J. 1975, 15(1), 17-20

(73) Patentinhaber: **Müller, Heinz Joachim,**
**Schwibbogenplatz 2, D-8900 Augsburg (DE)**

(72) Erfinder: **Müller, Heinz Joachim,**
**Schwibbogenplatz 2, D-8900 Augsburg (DE)**

(74) Vertreter: **Struif, Bernward, Dipl.- Chem. Dr.,**
**Patentanwaltsbüro Tiedtke, Bühling, Kinne**
**Grupe, Pellmann, Grams, Struif, Winter, Roth**
**Bavariaring 4, D-8000 München 2 (DE)**

## Beschreibung

Die Erfindung betrifft ein Verfahren zur Bestimmung von Nitrat in Wasser.

In den letzten Jahren ist die Nitratkonzentration im Oberflächen- und im Grundwasser infolge einer Überdüngung in der Landwirtschaft ständig gestiegen. Aus der chemischen Wasserstatistik der Wasserwerke in der Bundesrepublik Deutschland und West-Berlin (bearbeitet von Dr. Gerhard Giebler, R. Oldenbourg Verlag, München, 1959) ergibt sich, daß die Nitratkonzentration im Oberflächen- und im Grundwasser in den meisten Fällen 50 mg/l erreicht oder sogar überschreitet. Nach Angaben der Weltgesundheitsorganisation soll die Nitratkonzentration im Trinkwasser jedoch 50 mg/l nicht überschreiten, da in diesem Fall die Gefahr besteht, daß bei Kindern Methämoglobinämie verursacht werden kann. Nach neueren Erkenntnissen kann Nitrat in Verbindung mit gewissen Aminen im Verdauungstrakt Nitrosamin bilden, das als stark krebserregend einzustufen ist. Es ist infolgedessen notwendig, die Nitratkonzentration im Wasser kontinuierlich zu überwachen.

Es sind zahlreiche Methoden zur Bestimmung von Nitrat im Wasser bekannt geworden. Die meisten Bestimmungen beruhen auf Farbreaktionen, die entweder eine Reduktion des Nitrats zu Nitrit und anschließende Diazotierung zu Azofarbstoffen oder eine direkte Reaktion des Nitrats mit p-Fluorphenol einschließen. Die letztgenannte fotometrische Bestimmung mittels p-Fluorphenol hat Eingang in die deutschen Einheitsverfahren gefunden, jedoch ist diese Bestimmung sehr aufwendig und erfordert eine genaue Überwachung der Chemikaliendosierung. Für eine rasche und kontinuierliche Bestimmung des Nitrats im Wasser ist diese Methode wenig geeignet.

Ferner ist es bekannt, das Nitrat durch direkte Reaktion mit Brucin zu bestimmen. Diese Bestimmungsmethode ist jedoch nur unter strengen Vorsichtsmaßnahmen durchführbar, da Brucin ein starkes Gift ist. Zur kontinuierlichen Überwachung des Nitrats im Wasser ist eine solche Methode ungeeignet.

Ferner sind in den letzten Jahren ionensensitive Elektroden bekannt geworden, die eine selektive Bestimmung des Nitrations im Wasser erlauben. So ist in der Zeitschrift Fresenius Z. Anal. Chem. 297, Seiten 414 bis 418 (1979) eine nitrationen-selektive Elektrode auf Basis von Kupfer(I)-Neocuproinkomplex beschrieben. Es hat sich jedoch herausgestellt, daß solche ionenselektiven Elektroden in erheblichem Maße störanfällig sind, so daß sie nicht zur kontinuierlichen Nitratbestimmung im Wasser zu empfehlen sind.

Es ist auch bekannt, daß das Nitrat im UV-Bereich bei 210 nm ein ausgeprägtes Absorptionsmaximum zeigt, das generell für eine quantitative Bestimmung des Nitrats herangezogen werden könnte. Da jedoch Oberflächen-, Grund- und Abwasser eine Vielzahl von anorganischen und organischen Wasserinhaltsstoffen besitzt, die im UV-Bereich absorbieren und damit die Nitrat-Absorption überlagern, ist bisher kein Verfahren bekannt geworden, bei dem eine reproduzierbare Bestimmung des Nitrats über die UV-Absorption erfolgt.

Aus der DE-OS-24 60 078 sind Hydrierkatalysatoren bekannt, die mindestens ein Metall der Platingruppe wie z. B. Pt und/oder Pd und mindestens ein Metall der Kupfergruppe wie z. B. Cu enthalten und für die selektive Hydrierung von Säurechloriden zu den entsprechenden Aldehyden und für die stereospezifische Hydrierung zahlreicher Verbindungen wie z. B. von Steroiden geeignet sind. Diese bekannten Hydrierkatalysatoren werden hergestellt, indem man die Metallionen aus einer Lösung, die lösliche Salze des Metalls der Platingruppe wie z. B. Hexachloroplatinsäure oder Palladiumchlorid und des Metalls der Kupfergruppe wie z. B. Kupferchlorid enthält, vorzugsweise in Gegenwart eines Katalysatorträgers wie z. B. Aktivkohle oder Aluminiumoxid, der mit der Lösung imprägniert wird, gemeinsam abscheidet und - z. B. mit Wasserstoff, Natriumborhydrid oder Ameisensäure - bei z. B. 0 bis 100 C reduziert. Auf den Katalysatorträger werden vorzugsweise 0,5 bis 10 Gew.-% des Metalls der Platingruppe und 0,05 bis 10 Gew.-% des Metalls der Kupfergruppe aufgebracht.

Aus der US-PS-2 967 835 ist ein Verfahren zur Herstellung eines Hydrierungskatalysators, der beispielsweise für die Hydrierung von Nitroverbindungen zu den entsprechenden Aminen und insbesondere für die Hydrierung von 1,4-Butindiol zu 1,4-Butendiol geeignet ist, bekannt, bei dem eine wäßrige Lösung, die ein Kupfersalz und metallisches Palladium, vorzugsweise auf einem Katalysatorträger wie z. B. Aktivkohle oder Aluminiumoxid, enthält, mit einem Reduktionsmittel wie z. B. Hydrazin behandelt wird, um auf dem Palladium in situ metallisches Kupfer (vorzugsweise 5 bis 30 Teile Kupfer je Teil Palladium) auszufällen.

Der Erfindung liegt die Aufgabe zugrunde, ein Verfahren zur Bestimmung von Nitrat in Wasser bereitzustellen, bei dem zur Bildung einer Blindprobe das Nitrat selektiv reduziert werden kann, ohne daß Wasserinhaltsstoffe mitreduziert werden, so daß eine rasche und empfindliche Bestimmung des Nitrats in Wasser insbesondere im kontinuierlichen Betrieb über die UV-Absorption möglich ist.

Diese Aufgabe wird dadurch gelöst, daß die Nitratextinktion einer Wasserprobe gegenüber einer Blindprobe im UV-Bereich bei 210 nm bestimmt wird, wobei als Blindprobe ein Teil der Wasserprobe eingesetzt wird, dessen Nitrat in Gegenwart von Wasserstoff unter Verwendung eines Katalysators reduziert wurde, der durch Reduktion einer Lösung eines Salzes eines Übergangsmetalls der Gruppe VIII des Periodensystems und eines Salzes eines Übergangsmetalls der Kupfergruppe in Gegenwart einer Säure erhalten worden ist.

Der Katalysator, der in dem erfindungsgemäßen Verfahren verwendet wird, ist in der Lage, das Nitrat in Wasser in Gegenwart von Wasserstoff selektiv zu reduzieren, ohne daß beispielsweise Sulfat zu Sulfid reduziert wird, was den Katalysator vergiften würde. Das erfindungsgemäße Verfahren kann zur Bestimmung von Nitrat in Leitungs-, Grund-, Oberflächen- und Abwasser angewandt werden.

Der Katalysator wird nachstehend im einzelnen näher erläutert.

Das Übergangsmetall der Gruppe VIII des Periodensystems ist vorzugsweise ein Übergangsmetall der Platingruppe, insbesondere Platin oder Palladium. Bei der Herstellung des Katalysator wird als Salz des Übergangsmetalls der Platingruppe vorzugsweise $M_2PtX_6$, $M_2PdX_6$, $PdX_2$ oder $PtX_2$ eingesetzt, wobei M ein Alkalimetall, Ammonium oder Wasserstoff und X ein Halogen ist. Ein besonders geeignetes Übergangsmetallsalz ist Ammoniumhexachloroplatinat.

Als Salz des Übergangsmetall Kupfergruppe kann ein Oxid, Carbonat, Nitrat oder Halogenid, insbesondere von Kupfer, eingesetzt werden.

Bei der Herstellung des Katalysators ist es wichtig, daß das Übergangsmetall der Gruppe VIII des Periodensystems und das Übergangsmetall der Kupfergruppe in einem bestimmten Molverhältnis eingesetzt wird. Bei Voruntersuchungen hat sich nämlich herausgestellt, daß das Nitrat zwar durch Raney-Nickel und ebenso durch Platin reduziert wird; jedoch wurde festgestellt, daß das im Wasser vorhandene Sulfat zum Sulfid reduziert wurde. Da Sulfid ein Katalysatorgift ist, wird der Katalysator aus Raney-Nickel nach kurzer Zeit inaktiv, so daß er für den vorliegenden Zweck ungeeignet ist. Ähnliche Ergebnisse wurden bei Vorversuchen mit Platin erhalten, bei der selbst in Anwesenheit von 10 mg/l Sulfat das Platin rasch inaktiviert wurde.

Aus den vorstehend genannten Gründen liegt das Übergangsmetall der Gruppe VIII des Periodensystems und das Übergangsmetall der Kupfergruppe im Katalysator geeigneterweise in einem Molverhältnis von 1 : 8 bis 8 : 1, vorzugsweis von 1 : 4 bis 4 : 1, vorhanden. Insbesondere liegt das Molverhältnis des Übergangsmetall der Gruppe VIII des Periodensystems zu dem Übergangsmetall der Kupfergruppe bei etwa 1 : 2 bis 1 : 4.

Bei der Reduktion der Übergangsmetallsalze zu dem Katalysator wird als Säure meist eine anorganische Säure, insbesondere Salzsäure oder Salpetersäure oder ein Gemisch dieser beiden Säuren, verwendet. Statt dem unmittelbaren Einsatz von Übergangsmetallsalzen können auch die Übergangsmetalle in elementarer Form eingesetzt werden, indem beispielsweise Platinschnitzel und Kupferdraht in Königswasser aufgelöst werden.

Bei der Herstellung des Katalysators erfolgt die Reduktion der Übergangsmetallsalze gleichzeitig, wobei als Reduktionsmittel ein solches verwendet wird, das in der Spannungsreihe unterhalb des Kupfers steht. Bevorzugte Reduktionsmittel sind beispielsweise Zinkstaub oder eine Hydrazinverbindung.

Der Katalysator wird durch Reduktion bei einer Temperatur von 10 bis 150°C, insbesondere bei 40 bis 80°C erhalten.

Gemäß einer bevorzugten Ausführungsform wird ein auf einen Träger aufgebrachter Katalysator verwendet, der durch Reduktion in Gegenwart des Trägers erhalten worden ist Zweckmäßigerweise wird die Lösung der Übergangsmetallsalze in Gegenwart eines porösen Trägers zunächst einem Unterdruck ausgesetzt, so daß dieser mit der Lösung getränkt wird. Dieser Verfahrensschritt kann beispielsweise in einem Vakuumexsikkator durchgeführt werden. Danach wird die Lösung mit dem so präparierten Träger auf Atmosphärendruck zurückgebracht, worauf die eingetragenen Übergangsmetallsalze reduziert werden.

Als poröse Träger kommen keramische Materialien, wie Aluminiumoxid, Magnesiumoxid, Kieselgel oder Kieselgur, Aktivkohle oder Blähton in Betracht. Der Träger kann in Form von Berlsätteln oder Raschigringen vorliegen so daß der auf den Träger aufgebrachte Katalysator in Füllkörperkolonnen eingesetzt werden kann.

Nach einer weiteren Ausführungsform kann der Träger auch in Form einer Elektrode vorliegen. die zur Wasserstoffentwicklung benötigt wird und auf die eine Beschichtung aus dem Katalysator aufgebracht worden ist. In diesem Falle kann die Elektrode aus irgendeinem geeigneten Material, beispielsweise aus Kohle, bestehen.

Das erfindungsgemäße Verfahren gestattet eine rasche und selektive Bestimmung des Nitrats im Wasser, ohne daß eine erhebliche Manipulation der Wasserprobe durchgeführt wird und wobei weitgehend eine Chemikalienzugabe vermieden werden kann. Das erfindungsgemäße Verfahren eignet sich insbesondere für die kontinuierliche Nitratbestimmung und damit zur kontinuierlichen Überwachung der Wasserqualität.

Das erfindungsgemäße Verfahren wird unter Bezugnahme auf die beiliegenden Figuren 1 bis 3 näher erläutert.

Fig. 1 zeigt das UV-Spektrum des Nitrats bei verschiedenen Nitratkonzentrationen.

Fig. 2 zeigt die Abhängigkeit der Nitratextinktion von der Nitratkonzentration bei 210 nm.

Fig. 3 zeigt eine schematische Darstellung des Verfahrensablaufs.

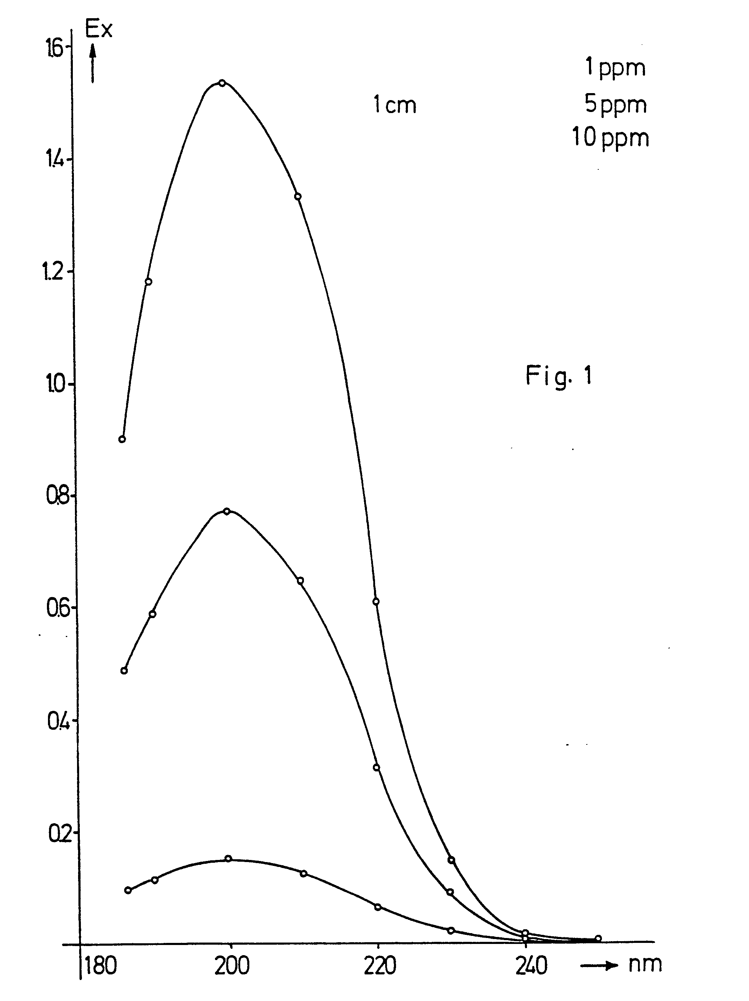

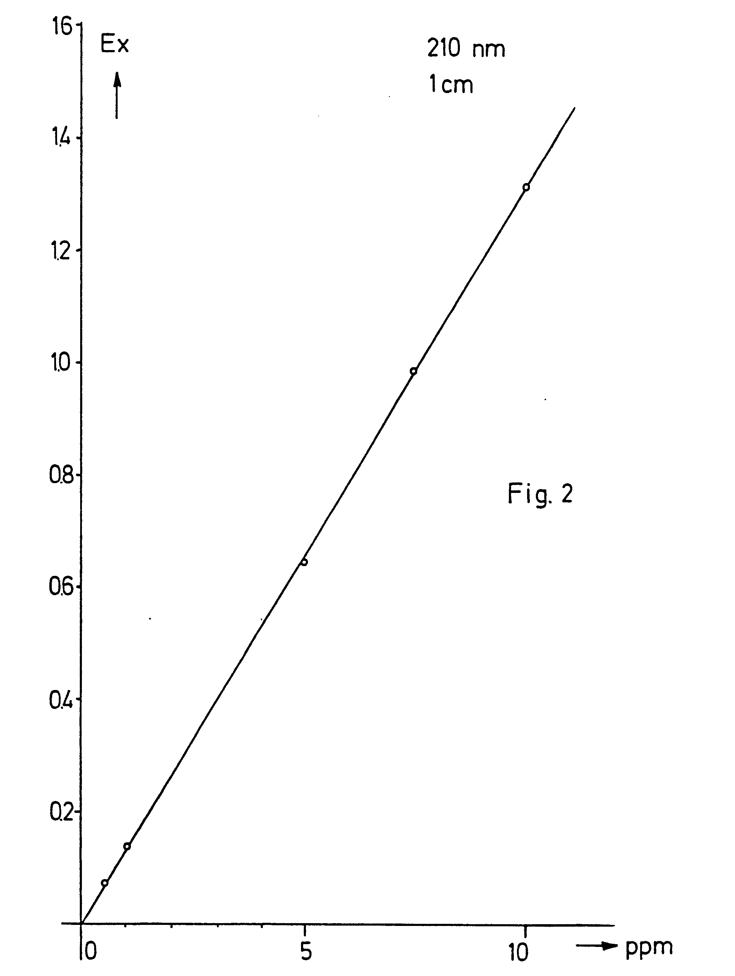

Wie aus Fig. 1 ersichtlich ist, zeigt das Nitrat im UV-Bereich, insbesondere bei 210 nm ein ausgeprägtes Absorptionsmaximum. Wie sich ferner aus Fig. 2 entnehmen läßt, ergibt sich ein exakt linearer Verlauf zwischen der Extinktion des Nitrats bei 210 nm und der Nitratkonzentration, so daß die Lichtschwächung des Nitrats im UV-Bereich für die quantitative Bestimmung des Nitrats im ppm-Bereich geeignet ist.

Durch eine selektive Reduktion des Nitrats in einem Teil der Wasserprobe wird eine Blindprobe erhalten, die sich von der zu messenden Wasserprobe lediglich dadurch unterscheidet, daß die Absorptionsbande des Nitrats als Folge der Nitratreduktion verschwunden ist, so daß eine exakt vergleichbare Blindprobe zur Verfügung steht. Die nach dem erfindungsgemäßen Verfahren erhaltenen Nitrat-Meßwerte waren gut reproduzierbar, was durch Vergleich mit einer modifizierten Brucin-Meßmethode überprüft wurde.

Bei der Durchführung der Nitratreduktion kann der Wasserstoff aus einer separaten Quelle geliefert werden. Vorzugsweise wird der Wasserstoff jedoch elektrolytisch aus dem Wasser gewonnen, so daß keine separate Chemikaliendosierung erforderlich ist.

Der zur Nitratreduktion eingesetzte Katalysator kann als Katalysatorschwamm in einem Wirbelbett vorliegen, durch das der zu reduzierende Teil der Wasserprobe geleitet wird. Gemäß einer bevorzugten Ausführungsform der Erfindung wird die Nitratreduktion mit einem auf einen Träger aufgebrachten Katalysator durchgeführt, der beispielsweise in eine Füllkörperkolonne eingefüllt ist.

Durch eine solche mit dem auf einen Träger aufgebrachten Katalysator beschickte Füllkörperkolonne wird dann zur Bildung der Blindprobe der zu reduzierte Teil der Wasserprobe hindurchgeleitet. Daneben kann die Reduktion auch durchgeführt werden, indem eine für die Wasserstoffentwicklung benötigte Elektrode eingesetzt wird, die mit dem Katalysator beschichtet ist.

Vorzugsweise wird zur Bildung der Blindprobe die Wasserprobe im Gegenstrom zum Wasserstoff geführt. Hierbei sollte die Wasserprobe bis zu 10 Minuten lang, insbesondere 2 bis 6 Minuten lang in Gegenwart von Wasserstoff und Katalysator reduziert werden. Die Nitratreduktion kann bei 5 bis 30°C, vorzugsweise bei Raumtemperatur, durchgeführt werden.

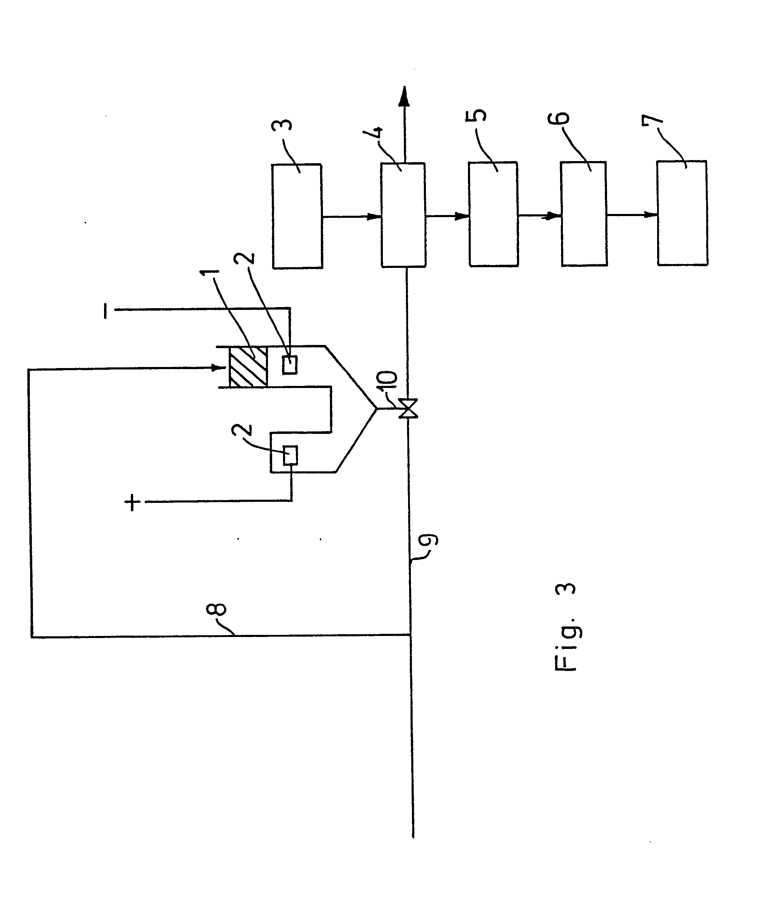

Eine zweckmäßige Ausführungsform zur Durchführung des erfindungsgemäßen Verfahrens ist in Fig. 3 gezeigt.

Hierbei wird eine Wasserprobe über die Leitung 9 in eine Küvette 4 geleitet, die sich innerhalb eines Fotometers befindet. Ein Teil der Wasserprobe wird über die Leitung 8 zu einer Füllkörperkolonne 1 geleitet, die mit dem auf einen Träger aufgebrachten Katalysator gefüllt ist. Der zu reduzierende Teil der Wasserprobe strömt durch die Füllkörperkolonne, beispielsweise mit einer Geschwindigkeit von 100 bis 300 ml/h, wobei gleichzeitig Wasserstoff im Gegenstrom aufsteigt, der elektrolytisch über die Elektroden 2 erzeugt wird. Die eingeführte Wasserstoffmenge beträgt zweckmäßigerweise etwa 40 bis 80 ml/min. Der auf diese Weise reduzierte Teil der Wasserprobe strömt über die Leitung 10 in eine Quarzküvette 4 innerhalb des Fotometers und dient hier als Blindprobe. Das Fotometer enthält neben den Quarzküvetten 4 eine UV-Lampe 3, einen Detektor 5, einer Speicher 6 und ein Anzeigegerät 7. Als Quarzküvetten können Durchflußküvetten verwendet werden, so daß eine kontinuierliche Betriebsweise möglich ist.

**Beispiel 1**

444 mg $(NH_4)_2PtCl_6$ und 269 mg $CuCl_2$ werden in einer Mischung aus 40 ml 37%iger Salzsäure und 80 ml Wasser gelöst. Unter Rühren mittels eines Magnetrührers werden 2 g Zn-Staub hinzugegeben. Nach Beendigung der Gasentwicklung wird das Zementat chloridfrei gewaschen und als Katalysator im Wirbelbett verwendet.

**Beispiel 2**

444 mg $(NH_4)_2 PtCl_6$ und 269 mg $CuCl_2$ wurden in einer Mischung aus 40 ml Salzsäure (37%ig) und 80 ml Wasser gelöst. 10 g Blähton wurden im Vakuum (Vakuumexsikkator) mit dieser Lösung getränkt. Nach dem Entspannen auf Atmosphärendruck erfolgte der Eintrag des präparierten Blähtons in eine Mischung aus 40 ml 37%iger Salzsäure und 80 ml Wasser. Anschließend wurde durch Zugabe von 2 g Zn-Staub reduziert. Der auf den Träger aufgebrachte Katalysator wurde in einer Füllkörperkolonne eingesetzt.

**Beispiel 3**

1 g Platinschnitzel und 1,5 g Kupferdraht wurden in Königswasser gelöst. Anschließend wurde bis fast zur Trockne abgeraucht und mit 37%iger Salzsäure aufgenommen und wiederum abgeraucht. Dieser Vorgang wurde so lange wiederholt, bis keine nitrosen Dämpfe mehr auftraten, was nach viermaligen Abrauchen der Fall war. Nach dem Lösen des Rückstands in einer Mischung aus 40 ml 37%iger Salzsäure und 80 ml Wasser erfolgte die Reduktion durch 2 g Zn-Staub.

**Beispiel 4**

Im nachfolgenden Beispiel wurde der Abbau von Nitrat in Abhängigkeit vom Molverhältnis Platin/Kupfer im Zementat untersucht. Als Modellwasser diente Leitungswasser, das 15 mg/l Sulfat enthielt und mit Natriumnitrat auf 10 mg/l Nitrat dotiert war. In einer 200 ml fassenden Waschflasche wurden chargenweise je 100 ml Wasser im Beisein des Katalysators der durch eine Natriumnitratlösung von dem durch die Herstellungsbedingungen anwesenden Wasserstoff befreit wurde 5 Minuten lang unter Rühren mit Wasserstoff begast. Die Zufuhr des Wasserstoffgases betrug ca. 60 ml/min. Die Abnahme des Nitrats wurde über die Extinktion im UV-Bereich bei 210 nm (Ex 210) sowie als Referenz über eine modifizierte Brucin-Methode bestimmt.

**Tabelle I**

| Molverhältnis Pt:Cu | 1. Charge | 2. Charge | 3. Charge | NO₃-Ex. 210 c/c° | NO₃-Brucin c/c° |
|---|---|---|---|---|---|
| 1:2 | 0,025 | 0,033 | 0,012 | 0,01 | 0,03 |
| 2:1 | 0,050 | 0,220 | 0,750 | 0,57 | 0,54 |
| 3:1 | 0,013 | 0,350 | 0,980 | 0,74 | 0,70 |
| 3:2 | 0,018 | 0,195 | 0,630 | 0,48 | 0,52 |
| 1:3 | 0,065 | 0,078 | 0,069 | 0,05 | 0,04 |

Aus der vorstehenden Tabelle ist es ersichtlich, daß ein Katalysator mit einem Molverhältnis von Platin zu Kupfer von 1:2 bzw. 1:3 die besten Ergebnisse liefert, da hiermit das Nitrat praktisch bis zur Nachweisgrenze abgebaut wird. Bei den drei anderen Platin/Kupferkatalysatoren wird ein zunehmend unvollständiger Abbau des Nitrats beobachtet, was darauf zurückzuführen ist, daß durch diese Katalysatoren das vorhandene Sulfat zum Sulfid reduziert wird, was den Katalysator vergiftet. Aus den beiden letzten Spalten der Tabelle I wird deutlich, daß die über die Brucin-Methode erhaltenen Ergebnisse mit den nach dem erfindungsgemäßen Verfahren erhaltenen Ergebnissen gut vergleichbar sind.

**Beispiel 5**

Im vorliegenden Versuch wurde das Langzeitverhalten des auf einem Träger aufgebrachten Katalysators in einer Füllkörperkolonne untersucht. Eine Bürette mit eingeführter Fritte (Ø = 2 cm) wurde bis zu einer Füllhöhe von 50 cm mit einem nach dem Beispiel 2 hergestellten Trägerkatalysator gefüllt und von unten mit ca. 60 ml H₂/min begast. Im Gegenstrom wurde Leitungswasser, dotiert mit 10 mg Nitrat/l, durch die Schüttung bei einer Strömungsgeschwindigkeit bei 200 ml/h geführt.

**Tabelle II**

| Dauer h | Ex. 210 nm | c/c° Ex. 210 nm | c/c° Brucin |
|---|---|---|---|
| 4 | 0,033 | 0,03 | 0,03 |
| 8 | 0,068 | 0,05 | |
| 12 | 0,012 | 0,01 | |
| 16 | 0,055 | 0,04 | 0,06 |
| 20 | 0,045 | 0,04 | |
| 24 | 0,063 | 0,05 | |
| 28 | 0,035 | 0,03 | 0,10 |
| 32 | 0,048 | 0,04 | |
| 36 | 0,042 | 0,03 | |
| 40 | 0,059 | 0,05 | 0,05 |

Aus den vorstehenden Versuchsergebnissen wird deutlich, daß das Nitrat auch bei längerer Behandlungsdauer durch den Katalysator praktisch vollständig reduziert wird, da die angegebenen Extinktionswerte an der Nachweisgrenze liegen. Außerdem ergibt sich aus den beiden letzten Spalten der Tabelle II eine gute Übereinstimmung zwischen der Meßergebnissen, die einerseits nach dem erfindungsgemäßen Verfahren und andererseits nach der modifizierten Brucin-Methode erhalten wurden.

**Vergleichsversuch 1**

In diesem Vergleichsversuch wurden die katalytischen Eigenschaften von Raney-Nickel untersucht. Als Modelllösung diente destilliertes Wasser, das 10 mg/l Nitrat enthielt. Nach Konktaktzeiten von 30 bis 60 Minuten war Nitrat nicht mehr nachweisbar. Weiterhin wurde beobachtet, daß selbst bei Begasung mit Wasserstoff die Reduktionskraft des Materials nachließ.

Bei Versuchen mit Leitungswasser stellte sich jedoch heraus, daß zum einen das Raney-Nickel schneller inaktiviert wurde, zum anderen größere Mengen Raney-Nickel zur Reduktion der gleichen Nitratmenge als im destillierten Wasser erforderlich waren. Die Ursache für die Inaktivierung lag darin, daß in dem Leitungswasser Sulfat vorhanden war, das zum Sulfid reduziert wurde, und dieses als Katalysatorgift wirkte.

**Vergleichsversuch 2**

Es wurden ähnlich Versuche wie im Vergleichsbeispiel 1 unter Verwendung von Platinschwamm durchgeführt. Hierbei wurden im wesentlichen dieselben Ergebnisse wie bei Verwendung von Raney-Nickelerhalten mit der Ausnahme, daß in Abwesenheit von Sulfat keine Inaktivierung eintrat. Das heißt daß bei Begasung mit Wasserstoff eine kontinuierliche Reduktion des Nitrats stattfindet. Bei Anwesenheit von Sulfat selbst bei Konzentrationen von etwa 10 mg/l wird das Platin jedoch rasch inaktiviert.

**Patentansprüche**

1. Verfahren zur Bestimmung von Nitrat in Wasser, dadurch gekennzeichnet, daß die Nitratextinktion einer Wasserprobegegenüber einer Blindprobe im UV-Bereich bei 210 nm bestimmt wird, wobei als Blindprobe ein Teil der Wasserprobe eingesetzt wird, dessen Nitrat in Gegenwart von Wasserstoff unter Verwendung eines Katalysators reduziert wurde, der durch Reduktion einer Lösung eines Salzes eines Übergangsmetalls der Gruppe VIII des Periodensystems und eines Salzes eines Übergangsmetalls der Kupfergruppe in Gegenwart einer Säure erhalten worden ist.

2. Verfahren nach Anspruch 1, dadurch gekennzeichnet, daß das Übergangsmetall der Gruppe VIII des Periodensystems ein Übergangsmetall der Platingruppe, insbesondere Pt oder Pd, ist.

3. Verfahren nach Anspruch 1 oder 2, dadurch gekennzeichnet, daß das Salz des Übergangsmetalls der Platingruppe $M_2PtX_6$, $M_2PdX_6$, $PtX_2$ oder $PdX_2$ ist, wobei M ein Alkalimetall, Ammonium oder Wasserstoff ist und X ein Halogen ist.

4. Verfahren nach Anspruch 3, dadurch gekennzeichnet, daß M Ammonium und X Chlor ist.

5. Verfahren nach Anspruch 1, dadurch gekennzeichnet, daß das Salz des Übergangsmetalls der Kupfergruppe ein Oxid, Carbonat, Nitrat oder Halogenid von Kupfer ist.

6. Verfahren nach einem der vorhergehenden Ansprüche, dadurch gekennzeichnet, daß das Übergangsmetall der Gruppe VIII des Periodensystems und das Übergangsmetall der Kupfergruppe in einem Molverhältnis von 1:8 bis 8:1, insbesondere von 1:4 bis 4:1, vorhanden sind.

7. Verfahren nach Anspruch 6, dadurch gekennzeichnet, daß das Molverhältnis des Übergangsmetalls der Gruppe VIII des Periodensystems zu dem Übergangsmetall der Kupfergruppe bei etwa 1:2 bis 1:4 liegt.

8. Verfahren nach einem der vorhergehenden Ansprüche, dadurch gekennzeichnet, daß die Säure eine anorganische Säure, insbesondere Salzsäure oder Salpetersäure oder ein Gemisch dieser beiden Säuren, ist.

9. Verfahren nach einem der vorhergehenden Ansprüche, dadurch gekennzeichnet, daß ein Katalysator verwendet wird, der durch Reduktion mit einem Reduktionsmittel erhalten worden ist, das in der Spannungsreihe unterhalb des Kupfers steht.

10. Verfahren nach Anspruch 9, dadurch gekennzeichnet, daß das Reduktionsmittel Zinkstaub oder eine Hydrazinverbindung ist.

11. Verfahren nach einem der vorhergehenden Ansprüche, dadurch gekennzeichnet, daß ein Katalysator verwendet wird, der durch Reduktion bei 10°C bis 150°C, insbesondere bei 40°C bis 80°C, erhalten worden ist.

12. Verfahren nach einem der vorhergehenden Ansprüche, dadurch gekennzeichnet, daß ein auf einen Träger aufgebrachter Katalysator verwendet wird, der durch Reduktion in Gegenwart des Trägers erhalten worden ist.

13. Verfahren nach Anspruch 12, dadurch gekennzeichnet, daß ein Katalysator verwendet wird, der erhalten worden ist, indem man die Lösung der Übergangsmetallsalze in Gegenwart eines porösen Trägers einem Unterdruck aussetzt, den so präparierten Träger auf Atmosphärendruck zurückbringt und die eingetragenen Übergangsmetallsalze reduziert.

14. Verfahren nach Anspruch 12 oder 13, dadurch gekennzeichnet, daß der poröse Träger aus keramischen Materialien, Aktivkohle oder Blähton besteht.

15. Verfahren nach Anspruch 14, dadurch gekennzeichnet, daß der Träger in Form von Berlsätteln oder Raschigringen vorliegt.

16. Verfahren nach Anspruch 12, dadurch gekennzeichnet, daß der Träger in Form einer Elektrode vorliegt, auf die eine Beschichtung aus dem Katalysator aufgebracht worden ist.

17. Verfahren nach Anspruch 16, dadurch gekennzeichnet, daß die Elektrode aus Kohle besteht.

18. Verfahren nach Anspruch 1, dadurch gekennzeichnet, daß der Wasserstoff aus einer separaten Quelle geliefert oder elektrolytisch aus dem Wasser gewonnen wird.

19. Verfahren nach Anspruch 1 oder 18, dadurch gekennzeichnet, daß das Nitrat bei 5°C bis 30°C, insbesondere bei Raumtemperatur, reduziert wird.

20. Verfahren nach einem der vorhergehenden Ansprüche, dadurch gekennzeichnet, daß zur Bildung der Blindprobe ein Teil der Wasserprobe durch ein Wirbelbett des Katalysators geleitet wird.

21. Verfahren nach Anspruch 12, dadurch gekennzeichnet, daß zur Bildung der Blindprobe ein Teil der Wasserprobe durch eine Füllkörperkolonne geleitet wird, die mit dem auf einen Träger aufgebrachten Katalysator beschickt ist.

22. Verfahren nach Anspruch 16, dadurch gekennzeichnet, daß man zur Bildung der Blindprobe einen Teil der Wasserprobe mit einer Elektrode für die Wasserstoffentwicklung in Berührung bringt, die mit dem Katalysator

**0 074 477**

beschichtet ist.

23. Verfahren nach einem der Ansprüche 1 bis 15 und 18 bis 21, dadurch gekennzeichnet, daß man zur Bildung der Blindprobe die Wasserprobe im Gegenstrom zum Wasserstoff führt.

24. Verfahren nach einem der vorhergehenden Ansprüche, dadurch gekennzeichnet, daß man zur Bildung der Blindprobe einen Teil der Wasserprobe bis zu 10 Minuten lang, insbesondere 2 bis 6 Minuten lang, reduziert.

**Claims**

1. A method for the determination of nitrate in water, characterized in that the nitrate extinction of the water sample as against a blank is derermined in the UV range at 210 nm, where as blank a part of the water sample is used, whose nitrate was reduced in the presence of hydrogen by means of a catalyst, which was obtained through reduction of a solution of a salt of a transition metal of group VIII of the periodic system and a salt of a transition metal of the copper group in the presence of an acid.

2. The method in accordance with claim 1, characterized in that the transition metal of group VIII of the periodic system is a transition metal of the platinum group, in particular Pt or Pd.

3. The method in accordance with claim 1 or 2, characterized in that the salt of the transition metal of the platinum group is $M_2PtX_6$, $M_2PdX_6$, $PtX_2$ or $PdX_2$, where M is an alkali metal, ammonium or hydrogen, and X is a halogen.

4. The method in accordance with claim 3, characterized in that M is ammonium and X is chlorine.

5. The method in accordance with claim 1, characterized in that the salt of the transition metal of the copper group is an oxide, carbonate, nitrate or halide of copper.

6. The method in accordance with one of the preceding claims, characterized in that the transition metal of group VIII of the periodic system and the transition metal of the copper group are present in a molar ratio of 1 : 8 to 8 : 1, in particular 1 : 4 to 4 : 1.

7. The method in accordance with claim 6, characterized in that the molar ratio of the transition metal of group VIII of the periodic system to the transition metal of the copper group is around 1 : 2 to 1 : 4.

8. The method in accordance with one of the preceding claims, characterized in that the acid is an inorganic acid, in particular hydrochloric acid or nitric acid or a mixture of these two acids.

9. The method in accordance with one of the preceding claims, characterized in that a catalyst is used which was obtained by reduction with a reducing agent, which in the electrochemical series is below copper.

10. The method in accordance with claim 9, characterized in that the reducing agent is zinc dust or a hydrazine compound.

11. The method in accordance with one of the preceding claims, characterized in that a catalyst is used, which was obtained through reduction at 10°C to 150°C, in particular at 40°C to 80°C.

12. The method in accordance with one of the preceding claims, characterized in that a catalyst applied on a substrate is used, which catalyst was obtained through reduction in the presence of the substrate.

13. The method in accordance with claim 12, characterized in that a catalyst is used, which was obtained by exposing the solution of the transition metal salts to a partial vacuum in the presence of a porous substrate, by bringing back the substrate thus prepared to atmospheric pressure, and by reducing the entered transition metal salts.

14. The method in accordance with claim 12 or 13, characterized in that the porous substrate consists of ceramic materials, active charcoal or expanded clay.

15. The method in accordance with claim 14, characterized in that the substrate is present in the form of Berl saddles or Raschig rings.

16. The method in accordance with claim 12, characterized in that the substrate is present in the form of an electrode, on which a coating from the catalyst has been applied.

17. The method in accordance with claim 16, characterized in that the electrode consists of carbon.

18. The method in accordance with claim 1, characterized in that the hydrogen is supplied from a separate source or is electrolytically obtained from water.

19. The method in accordance with claim 1 or 18, characterized in that the nitrate is reduced at 5°C to 30°C in particular at room temperature.

20. The method in accordance with one of the preceding claims, characterized in that for the formation of the blank a part of the water sample is passed through a fluid bed of the catalyst.

21. The method in accordance with claim 12, characterized in that for the formation of the blank a part of the water sample is passed through a packed column which is filled with the catalyst applied on a substrate.

22. The method in accordance with claim 16, characterized in that for the formation of the blank a part of the water sample is brought into contact with an electrode for the hydrogen development, which electrode is coated with the catalyst.

23. The method in accordance with one of claims 1 to 15 and 18 to 21, characterized in that for the formation of the blank the water sample is countercurrently supplied to the hydrogen.

24. The method in accordance with one of the preceding claims, characterized in that for the formation of the blank a part of the water sample is reduced for up to 10 minutes, in particular for 2 to 6 minutes.

7

**Revendications**

1. Procédé de mise en évidence de nitrate dans l'eau caractérisé par le fait que l'extraction du nitrate d'un échantillon d'eau par rapport à une analyse à blanc est déterminée à 210 nm dans les ultraviolets, l'analyse à blanc étant constituée par une partie de l'échantillon d'eau dont le nitrate est réduit en présence d'hydrogène au moyen d'un catalyseur qui est obtenu par réduction d'une solution d'un sel de métal transitoire du groups VIII de la classification périorique et d'un sel de métal transitoire du groupe du cuivre en présence d'un acide.

2. Procédé selon la revendication 1 caractérisé par le fait que le metal transitoire du groupe VIII de la classification périodique est un métal transitoire du groupe du platine, en particulier Pt ou Pd.

3. Procédé selon la revendication 1 ou 2 caractérisé par le fait que le sel du métal transitoire du groupe du platine est $M_2PtX_6$, $M_2 PdX_6$, $PtX_2$ ou $PdX_2$, où est un métal alcalin, et de l'amonium ou de l'hydrogène et X un halogène.

4. Procédé selon la revendication 3 caractérisé par le fait que M est de l'ammonium et X du chlore.

5. Procédé selon la revendication 1 caractérisé par le fait que le sel de métal transitoire du groupe du cuivre est un oxyde, un carbonate, un nitrate ou un halogènure de cuivre,

6. Procédé selon l'une des revendications précédentes, caractérisé par le fait que le métal transitoire du groupe VIII de la classification périodique et le métal transitoire du groupe du cuivre sont présents dans un rapport molaire de 1:8 à 8:1, en particulier de 1:4 à 4:1.

7. Procédé selon la revendication 6 caractérisé par le fait que le rapport molaire du métal transitoire du groupe VIII de la classification périodique au métal transitoire du groupe du cuivre se situe à environ 1:2 à 1:4.

8. Procédé selon l'une des revendications précédentes caractérisé par le fait que l'acide est un acide anorganique notamment l'acide chlorhydrique ou l'acide nitrique ou un mélange de ces deux acides.

9. Procédé selon l'une des revendications précédentes caractérisé par le fait que l'on utilise un catalyseur qui est obtenu par réduction au moyen d'un réducteur situé dans la classification électrochimique au dessous du cuivre.

10. Procédé selon la revendication 9 caractérisé par le fait que le réducteur est une poussière de Zinc ou un composé.

11. Procédé selon l'une des revendications précédentes caractérisé par le fait qu'on utilise un catalyseur obtenu par réduction vers 10° à 150°, notamment vers 40° à 80°C.

12. Procédé selon l'une des revendications précédentes caractérisé par le fait que on utilise un catalyseur rapporté sur un substrat, qui a été obtenu par réduction en présence du substrat

13. Procédé selon la revendication 12 caractérisé par le fait que on cultive un catalyseur obtenu en soumettant la solution des sels de métal transitoire en présence d'un substrat poreux à une dépression en ramenant le substrat ainsi préparé à la pression atmosphérique et en réduisant les sels de métal transitoire rapportés.

14. Procédé selon la revendication 12 ou 13 caractérisé par le fait que le substrat poreux est constitué par des matériaux céramiques, du charbon actif ou de l'argile expensé.

15. Procédé seion la revendication 14 caractérisé par le fait que le substrat rest sous forme de selles Berl ou d'anneaux Raschig.

16. Procédé selon la revendication 12 caractérisé par le fait que le substrat est sous forme d'électrode sur laquelle on a rapporté une couche de catalyseur.

17. Procédé selon la revendication 16 caractérisé par le fait que l'électrode est en charbon.

18. Procédé selon la revendication 1 caractérisé par le fait que l'hydrogène est fournie par une source séparée ou obtenue de l'eau par électrolyse.

19. Procédé selon la revendication 1 ou 18 caractérisé par le fait que le nitrate est réduit entre 5°C et 30°C, en particulier à la température ambiante.

20. Procédé selon l'une quelconque des revendications précédentes caractérisé par le fait que pour faire une analyse à blanc une partie de l'échantillon d'eau est amenée par un lit fluidisé au catalyseur.

21. Procédé selon la revendication 12 caractérisé par le fait, que pour faire l'analyse à blanc une partie de l'échantillon d'eau est amenée par un corps de remplissage qui est pourvu du catalyseur rapporté sur un substrat.

22. Procédé selon la revendication 16 caractérisé par le fait que pour faire l'analyse à blanc on amène une partie de l' échantillon d'eau en contact avec l'électrode pour le dégagement d'hydrogène, qui est recouverte par le catalyseur.

23. Procédé selon l'une des revendications 1 à 15 et 18 à 21 caractérisé par le fait que pour faire l'analyse à blanc on amène l'échantillon d'eau à contre courant de l'hydrogène.

24. Procédé selon l'une des revendications précédentes caractérisé par le fait que pour faire l'analyse à blanc, on réduit une partie de l'échantillon d'eau jusqu'à 10 minutes, notamment de 2 à 6 minutes.

0 074 477

Fig. 1

Fig. 2

Fig. 3